# EUROPÄISCHE PATENTSCHRIFT

(11) **EP 2 002 015 B1**
(45) Veröffentlichungstag und Bekanntmachung des Hinweises auf die Patenterteilung: **04.12.2013**
(21) Anmeldenummer: 07723808.7
(22) Anmeldetag: 30.03.2007
(51) Int. Cl.: C12Q 1/68

(54) **GEMISCH REVERSIBEL INHIBIERTER ENZYME**
MIXTURE OF REVERSIBLY INHIBITED ENZYMES
MÉLANGE D'ENZYMES À INHIBITION RÉVERSIBLE

(30) Priorität: 04.04.2006 DE 102006015960
(43) Veröffentlichungstag der Anmeldung: 17.12.2008
(73) Patentinhaber: Qiagen GmbH, 40724 Hilden (DE)
(72) Erfinder: ENGEL, Holger, 40724 Hilden (DE); LÖFFERT, Dirk, 40589 Düsseldorf (DE); MISSEL, Andreas, 40215 Düsseldorf (DE); PEIST, Ralf, 40723 Hilden (DE)
(74) Vertreter: polypatent BGL
(86) Internationale Anmeldenummer: PCT/EP2007/002866
(87) Internationale Veröffentlichungsnummer: WO 2007/115702

(56) Entgegenhaltungen:
- EP-A- 1 091 002
- EP-A2- 0 592 035
- US-A- 5 773 258
- LASSUS H ET AL: "Genetic alterations and protein expression of KIT and PDGFRA in serous ovarian carcinoma" BRITISH JOURNAL OF CANCER, Bd. 91, Nr. 12, 13. Dezember 2004 (2004-12-13), Seiten 2048-2055, XP002451758 ISSN: 0007-0920
- KIURU MAIJA ET AL: "Few FH mutations in sporadic counterparts of tumor types observed in hereditary leiomyomatosis and renal cell cancer families" CANCER RESEARCH, Bd. 62, Nr. 16, 15. August 2002 (2002-08-15), Seiten 4554-4557, XP002451759 ISSN: 0008-5472
- MALINEN E ET AL: "Comparison of real-time PCR with SYBR green I or 5'-nuclease assays and dot-blot hybridization with rDNA-targeted oligonucleotide probes in quantification of selected faecal bacteria" MICROBIOLOGY 01 JAN 2003 UNITED KINGDOM, Bd. 149, Nr. 1, 1. Januar 2003 (2003-01-01), Seiten 269-277, XP002451760 ISSN: 1350-0872

### SEQUENZPROTOKOLL

<110> Qiagen GmbH
<120> Gemisch reversibel inhibierter Enzyme
<130> P12925EPSQ
<140>
   <141>
<160> 6
<170> PatentIn Ver. 2.1
<210> 1
   <211> 21
   <212> DNA
   <213> Künstliche Sequenz
<220>
   <223> Beschreibung der künstlichen Sequenz: Primer
<400> 1
   agctgaaagt tgtggaagat g 21
<210> 2
   <211> 19
   <212> DNA
   <213> Künstliche Sequenz
<220>
   <223> Beschreibung der künstlichen Sequenz: Primer
<400> 2
   ggagctgtct acgcaagtc 19
<210> 3
   <211> 17
   <212> DNA
   <213> Künstliche Sequenz
<220>
   <223> Beschreibung der künstlichen Sequenz: Primer
<400> 3
   tgctccatga ggagaca 17
<210> 4
   <211> 21
   <212> DNA
   <213> Künstliche Sequenz
<220>
   <223> Beschreibung der künstlichen Sequenz: Primer
<400> 4
   gtgatccaga ctctgacctt t 21
<210> 5
   <211> 22
   <212> DNA
   <213> Künstliche Sequenz
<220>
   <223> Beschreibung der künstlichen Sequenz: Primer
<400> 5
   aagagaagct gaagaaaacc aa 22
<210> 6
   <211> 18
   <212> DNA
   <213> Künstliche Sequenz
<220>
   <223> Beschreibung der künstlichen Sequenz: Primer
<400> 6
   gtggagaggt gggtgtag 18

## Beschreibung

Die vorliegende Erfindung betrifft eine Zusammensetzung oder einen Kit, die/der sowohl mindestens ein durch chemische kovalente Modifikation reversibel inhibiertes Enzym, als auch mindestens eine durch nicht kovalente Bindung eines Polyanions reversibel inhibierte Polymerase enthält, die Verwendung eines Gemisches auf solche Weise reversibel inhibierter Enzyme zur Bearbeitung oder Vervielfältigung von Polynukleotiden sowie ein Verfahren zur spezifischen Amplifikation von DNA und / oder RNA unter gleichzeitiger Verwendung beider Arten von reversibel inhibierten Enzymen.

Bei der Bearbeitung von Polynukleotiden, z.B. dem Vervielfältigen, Schneiden, Trennen oder Ligieren von Polynukleotidsequenzen in *"in vitro"* Ansätzen werden die zu bearbeitenden Polymerstränge, bevorzugt DNA- oder RNA-Sequenzen, mit verschiedenen benötigten Komponenten wie z.B. geeigneten Puffern, Nukleinsäuremonomeren, Nukleinsäureoligomeren (z.B. Primer einer PCR) und den für die jeweils gewünschte Bearbeitung notwendigen bzw. geeigneten Enzymen und ggf. Coenzymen üblicherweise bei Raumtemperatur zusammengegeben. Anschließend wird der Ansatz auf die Temperatur erwärmt, bei der das/die eingesetzte(n) Enzyme ihr Temperaturoptimum hat/haben, oder es werden spezifische Temperaturzyklen durchlaufen. Die meisten Enzyme weisen zwar bezüglich ihrer Aktivität ein Temperaturoptimum auf, jedoch sind sie auch bei anderen Temperaturen, z.B. bei Raumtemperatur in gewissem Umfang katalytisch aktiv.

Mit Hilfe der Polymerase-Kettenreaktion (PCR) können bestimmte DNA-Sequenzen - auch aus einem Gemisch verschiedenster Sequenzen - durch Anwendung von sequenzspezifischen Oligonukleotiden, sogenannten "Primern", stark vervielfältigt werden. Diese auch als "Amplifikation" bezeichnete Vervielfältigung erfolgt mit Hilfe einer DNA-Polymerase. DNA-Polymerasen bilden aus einzelsträngiger DNA doppelsträngige DNA, indem sie an freie 3'-OH-Enden eines bereits an den Einzelstrang angelagerten DNA-Fragments die jeweils zum verbleibenden Einzelstrang komplemetären Basen anknüpfen. Da DNA-Polymerasen nicht sequenzspezifisch sind, findet ein "Auffüllen" eines einzelsträngigen Bereiches auf einer DNA-Sequenz immer dann statt, wenn eine partielle Doppelstrangbildung, z.B. durch Anlagerung eines DNA-Fragmentes an den Einzelstrang vorliegt. Bei der PCR werden zu der DNA, aus der eine bestimmte Sequenz amplifiziert werden soll, sequenzspezifische Primer gegeben, die zu der Sequenz komplementär sind, die vervielfältigt werden soll. Trotz der Sequenzspezifität kann es jedoch zu unspezifischer Bindung dieser Primer an Einzelstrang-DNA kommen, entweder in einem Bereich der DNA, der nicht von Interesse ist, oder auch durch Bildung von Primer-Dimeren, das heißt, durch Anlagerung eines Primers an einen anderen. Tritt eine solche unspezifische Bindung auf, füllt die DNA-Polymerase den verbleibenden Einzelstrang auf, so dass neben den gewünschten DNA-Sequenzen auch unerwünschte Sequenzen vervielfältigt werden.

Bei vielen PCR-Applikationen treten zwei häufige Fälle auf: zum einen steht nur sehr wenig Ausgangsmaterial zur Verfügung, zum anderen umfaßt das Ausgangsmaterial eine Vielzahl verschiedenster Sequenzen. Im ersten Fall kommt es aufgrund des anfänglichen Primerüberschusses häufig zu einer Primer-Dimerbildung und zur Amplifikation der so aneinandergelagerten "Primerdoppelstränge" anstelle des gewünschten Produkts, während im zweiten Fall eine nichtspezifische Anlagerung von Primern an andere als die Zielsequenz(en) zu einer Verstärkung "falscher" DNA-Sequenzen führen kann. In beiden Fällen führt dies zu reduzierter Sensitivität der Detektion bis hin zur Nicht-Detektion von vorhandenen Sequenzen, z. B. von Krankheitserrregern wie Viren und Bakterien.

Wegen der erhofften "Aussage" solcher PCR-Ansätze und der damit in Zusammenhang stehenden benötigten Sensitivität ist es insbesondere bei diagnostischen Anwendungen der PCR wichtig, dass die Bildung solcher Nebenprodukte so weit wie möglich minimiert wird. Um dieses Ziel zu erreichen, wurden verschiedene Methoden entwickelt, die heutzutage als "hot start PCR" allgemein bekannt ist. Bei einer dieser Methoden wurden ursprünglich alle Reagenzien auf wenigstens 72°C erhitzt, bevor die DNA-Polymerase zugegeben wird (Chou et al. (1992), Nucleic acid Res. 20:1717-1723). Da ein solches Vorgehen zwar die Spezifizität deutlich erhöhte, aber für umfangreichere Ansätze unpraktikabel war, wurde diese Methode dahingehend weiterentwickelt, dass DNA-Polymerasen so modifiziert wurden, dass sie bei Raumtemperatur inaktiv waren, jedoch durch das Aufheizen der PCR-Ansätze auf die Schmelztemperatur von doppelsträngiger DNA aktiviert wurden. Diese Enzyme werden also erst aktiv, wenn die Gefahr einer unspezifischen Bindung von einzelsträngigen DNA Primern aufgrund der angelegten Temperatur deutlich geringer ist als bei Raumtemperatur.

Beispiele für solche bei Raumtemperatur inaktiven, reaktivierbaren DNA-Polymerasen sind in der Literatur beschrieben, wobei zwei Arten unterschieden werden können.

Bei einem Typ handelt es sich um Enzyme, die durch eine chemische Modifikation, also eine kovalente Bindung einer chemischen Substanz an das Enzym ihre katalytische Aktivität verlieren, bis sie durch Erhitzen reaktiviert werden.

Die US Patente 5,677,152 und 5,773,258 von Birch et al. aus den Jahren 1997 bzw. 1998 beschreiben z.B. eine Inhibierung von thermostabilen DNA-Polymerasen durch reversible chemische Modifikation von Lysinresten der Enzyme mit Citraconsäureanhydid.

Das US Patent 6,183,998 von Ivanov et al. aus dem Jahr 2001 offenbart eine reversible chemische Modifikation thermostabiler DNA-Polymerasen durch die Verknüpfung mit einem Aldehyd, bevorzugt Formaldehyd.

Die chemische Modifikation der Enzyme lässt sich oftmals nicht durch eine einmalige kurzzeitige Temperaturerhöhung auf 95°C vollständig aufheben, da die vollständige Renaturierung in einem einzigen Schritt bereits zu einer thermischen Degradation des Template-Materials führen könnte. Zudem liegen die Empfehlungen der Hersteller bezüglich der Zeit für den initialen Aktivierungsschritt in der Regel unterhalb des Zeitraums, der für die vollständige Reaktivierung benötigt wird. Vielmehr wird eine zunehmende Menge der chemisch modifizierten Enzyme erst im Laufe der sich wiederholenden Temperaturzyklen einer PCR aktiviert. Somit kann der Fall eintreten, dass zu Anfang einer PCR nicht ausreichend DNA-Polymeraseakitivität im Ansatz vorliegt, um z.B. längere Templates vollständig aufzufüllen, bevor der nächste PCR-Zyklus beginnt. Dies kann unvollständiger Synthese des Doppelstrangs zu Kettenabbrüchen während der PCR führen, was bewirkt, dass im schlechtesten Fall nur Fragmente der gewünschten DNA-Sequenz amplifiziert werden.

Bei dem zweiten bekannten Typ der reversiblen Inhibierung von DNA-Polymerasen handelt es sich um eine nicht-kovalente Bindung eines Inhibitors an das Enzym. Auch in diesem Fall läßt sich die Inhibierung des Enzyms durch Erwärmen (z.B. zu Beginn einer PCR) aufheben.

In den US Patenten 5,338,671 (1994) und 5,587,287 (1996) beschreiben Scalice et al. einen Ansatz, in dem die Bindung von spezifischen Antikörpern an DNA-Polymerasen genutzt wird, um die Aktivität der jeweiligen Polymerase bei Raumtemperatur zu inhibieren. Aufgrund der Erwärmung der PCR-Proben beim Start der PCR wird der Antikörper denaturiert und die thermostabile Polymerase wird aktiv.

Gold et al. beschreiben in US 5,693, 502 (1997) und US 6,020,130 einsträngige Oligonucleotid-Liganden, sogenannte Aptamere, die bei Raumtemperatur eine hohe Affinität zu thermostabilen Taq und Tth Polymerasen haben, bei erhöhten Temperaturen jedoch nicht mehr an die Enzyme gebunden sind.

In dem US Patent 6,667,165 von Peters (2003) wird die Temperatur-abhängige und damit reversible Inhibition einer thermostabilen DNA-Polymerase durch eine Komplexierung mit Polyanionen beschrieben. Die eingesetzten Polyanionen sind nicht vom Nucleinsäuretyp, sondern stellen ein Monomer oder ein Oligomer von mehrfach negativ geladenen Verbindungen dar, wobei die negativen Ladungen auf Phosphat-, Sulfat- oder Carboxylgruppen zurückgehen.

Die durch eine nicht-kovalente Bindung inhibierten Enzyme liegen bei Raumtemperatur inaktiviert vor, werden jedoch bereits durch den initialen Erwärmungsschritt einer PCR nahezu vollständig aktiviert.

Der Nachteil der sofortigen vollständigen Aktivierung der durch nicht-kovalente Bindung inhibierten Enzyme ist insbesondere der, dass bei Vorliegen einer geringen Menge an zu amplifizierendem Ausgangsmaterial und daher einem hohen Überschuss an Primern, oder bei Primern, deren Sequenzen zueinander in einem gewissen Umfang komplementär sind (und die daher auch "aneinander passen") die oben beschriebene Problematik der unspezifischen Amplifikation oder der Dimerbildung nur um einen PCR-Zyklus verschoben ist, da es auch bei den erhöhten Temperaturen während einer PCR immer wieder in gewissem Umfang zu Anlagerungen der Primer aneinander oder an Sequenzbereiche kommt, zu denen die Primer nicht vollständig komplementär sind. Liegt gleichzeitig eine hohe Polymeraseaktivität im Ansatz vor, werden die "mismatches" toleriert und die Polymerase wird aktiv, bevor die nicht vollständig passende Sequenz wieder abgelöst werden kann. Es kommt zur Amplifikation unerwünschter Sequenzen.

Die europäische Patentanmeldung EP 1 091 002 offenbart eine Methode zur sequentiellen Aktivierung enzymatischer Aktivitäten, wobei eine erste thermostabile DNA Polymerase, welche durch einen Antikörper reversibel inhibiert ist, und eine zweite thermostabile DNA Polymerase, welche durch kovalente Modifikation mit z.B. Aldehyden oder Dicarbonsäureanhydriden reversibel inhibiert ist, in der gleichen Reaktion eingesetzt werden.

Weiter offenbaren die Arbeiten von Lassus et al. (British Journal of Cancer (2004) 91, 2048-2055) und Kiuru et al. (Cancer Research (2002) 62, 4554-4557) PCR-Protokolle, wobei der Reaktionsansatz Standardkomponenten und eine Mischung der DNA Polymerasen Platinum Taq (ein durch nicht-kovalente Bindung eines Antikörpers reversibel inhibiertes, durch Erwärmen aktivierbares, thermostabiles Enzym) und AmpliTaq Gold (ein chemisch modifiziertes, durch Erwärmen aktivierbares, thermostabiles Enzym) enthält.

Aufgabe der vorliegenden Erfindung war es eine Möglichkeit bereitzustellen die Ergebnisse bei der Bearbeitung oder Vervielfältigung von Polynucleotiden, insbesondere längerer Polynucleotidsequenzen zu verbessern, ohne dabei Einbußen bezüglich der Amplifikations-Spezifität in Kauf nehmen zu müssen.

Diese Aufgabe wird gelöst durch eine Zusammensetzung, die sowohl ein durch chemische Modifikation reversibel inhibiertes Enzym, als auch eine durch nichtkovalente Bindung eines Polyanions reversibel inhibierte Polymerase, oder ein Gemisch von diesen enthält, durch einen Kit, der beide solche Enzyme oder eine Mischung daraus enthält, wie auch durch eine Verwendung eines Gemisches solcher Enzyme und ein Verfahren unter gleichzeitiger Verwendung solcher Enzyme.

Die gleichzeitige Verwendung, bzw. das gleichzeitige Einsetzen der beiden auf verschiedene Art reversibel inhibierten Enzyme nebeneinander in demselben Ansatz, z.B. zur Bearbeitung oder Vervielfältigung von Polynucleotiden, bzw. das Bereitstellen beider Enzyme in einem Kit zur gemeinsamen Verwendung in einem Ansatz ist Gegenstand der vorliegenden Erfindung.

Der Vorteil der gleichzeitigen Verwendung eines Enzyms, das durch eine chemische Modifikation reversibel inhibiert wurde und einer Polymerase, die durch eine nicht-kovalente Bindung eines Polyanions reversibel inhibiert wurde, ist, dass sich die nichtkovalente Bindung schnell aufheben lässt und somit schnell eine zuvor definierbare Menge an aktivem Enzym bereitsteht, während die chemische Modifikation an dem Enzym nicht so schnell, aber dafür über die Zeit in zunehmendem Maße aufgehoben werden kann, so dass im Laufe einer Reaktion immer mehr Aktivität einer Enzyms zur Verfügung gestellt werden kann, ohne dass hierfür zusätzliche Bearbeitungsschritte oder Handlungen notwendig sind.

Im Prinzip kann der dieser Erfindung zugrunde liegende Ansatz in jeder Anwendung eingesetzt werden, bei der zu einem definierten Zeitpunkt eine bestimmte zuvor definierte Menge an enzymatischer Aktivität benötigt wird, die benötigte Menge an Enzymaktivität im Laufe der Zeit, z.B. über die Zeitdauer einer ablaufenden Reaktion jedoch zunimmt. Ein Beispiel hierfür ist die Amplifikation mittels PCR, wie weiter unten auch detaillierter beschrieben, ohne jedoch hierauf beschränkt zu sein. Auch im Falle, dass es von Vorteil ist, wenn eine Polymerase zu einem anderen Enzym zeitlich verschoben aktiv ist, kann der vorliegende Erfindungsansatz gewählt werden. Ein Beispiel hierfür ist die reverse Transkription und anschließende PCR der erhaltenen cDNA, oder das Schneiden und anschließende Ligieren von DNA. Der Vorteil, wenn beide Enzyme nebeneinander in einem Ansatz vorliegen ist, dass weniger Bearbeitungsschritte notwendig sind und damit deutlich effizienter gearbeitet werden kann, insbesondere wenn sehr viele Proben gleichzeitig bearbeitet werden müssen, aber auch z.B. die Kontaminationsgefahr der Proben deutlich reduziert werden kann.

Gemäß der Erfindung ist die Inhibiton der eingesetzten Enzyme reversibel. Die Art des Aufhebens bzw. Rückgängigmachens der Inhibition hängt von der Art der Modifikation der Enzyme ab. Die Art des Aufhebens oder Rückgängigmachens der Inhibition sollte so gewählt werden, dass die Aktivität des Enzyms zurückerhalten werden kann. Dies soll heißen, dass nicht nur die Modifikation oder Komplexierung durch nicht-kovalente Bindung des Enzyms aufgehoben wird, sondern auch, dass durch das Aufheben der Modifikation/Komplexierung wieder ein Enzym erhalten wird, das in der Lage ist die Reaktion zu katalysieren, die es vor der Modifikation/Komplexierung katalysieren konnte. In einer bevorzugten Ausführungsform der Erfindung lässt sich die Modifikation (kovalente Bindung) oder die Komplexierung (nicht-kovalente Bindung) des Enzyms durch Zufuhr von Wärme aufheben. In diesem Falle ist es vorteilhaft, wenn das Enzym thermostabil ist. Eine weitere Möglichkeit der Aufhebung ist durch Änderung des pH-Wertes im Ansatz. In diesem Falle sollte das Enzym die pH-Wert Änderung tolerieren, bzw. sollte die pH-Wert Änderung hin zu dem Bereich erfolgen, in dem das Enzym sein Aktivitätsoptimum aufweist.

In einer bevorzugten Ausführungsform der Erfindung handelt es sich bei den hier angesprochenen Enzymen um solche, die in der Lage sind Polynucleotide zu "bearbeiten" oder zu vervielfältigen. Unter dem Begriff "Polynucleotid" soll eine mehr als eine Base enthaltende Sequenz von Nucleotiden verstanden werden, bevorzugt eine Sequenz von wenigstens 8 Nucleotiden. Die Sequenz kann einsträngig oder doppelsträngig sein, oder (auch partiell) als Tripelhelix vorliegen. Es kann sich um DNA, RNA oder DNA-RNA-Hybride handeln.
Enzyme, die gemäß der vorliegenden Erfindung bevorzugt verwendet werden können sind DNA-Polymerasen, RNA-Polymerasen, Ligasen, reverse Transkriptasen und Restriktions-Endo- oder -Exonucleasen. Diese Enzyme können je nach gewünschter "Bearbeitung" der Polynucleotide kombiniert werden. Ganz besonders bevorzugt ist es, wenn die beiden eingesetzten Enzyme Polymerasen, insbesondere thermostabile Polymerasen sind, besonders bevorzugt thermostabile DNA-Polymerasen, die gemäß der Erfindung eingesetzt werden, um in einer PCR spezifische DNA-Sequenzen zu generieren und zu vervielfältigen.

Unter dem Begriff "thermostabil" ist zu verstehen, dass das entsprechende Enzym selbst unter Erwärmung bis auf 98°C seine Aktivität nicht (vollständig) verliert und im allgemeinen ein Aktivitätsoptimum im Bereich zwischen 40 °C und 90°C hat, bevorzugt im Bereich zwischen 50 °C und 80°C. Solche thermostabilen Enzyme sind in der Fachwelt allgemein bekannt und werden auch unter der Definition "thermostabil" auf dem Markt angeboten.

Unter dem Begriff "Polymerase" ist eine DNA- oder eine RNA-Polymerase zu verstehen, insbesondere eine DNA-Polymerase oder eine reverse Transkriptase.

DNA-Polymerasen, die durch chemische Modifikation oder durch nicht-kovalente Bindung eines Inhibitors reversibel inhibierbar sind, sind z.B. thermostabile DNA-Polymerasen aus den Arten Thermus, Pyrococcus, Thermococcus, Thermotoga, Pyrodictium und Thermosipho, bevorzugt Thermus aquaticus, Thermus thermophilus, Thermus flavus, Thermus filiformis, Pyrococcus furiosus, Pyrococcus woesei, Pyrococcus Spec. (Stamm KOD1), Pyrococcus spec. GB-D, Thermococcus litoralis , Thermococcus sp.9° N-7, Thermotoga maritima, Pyrococcus. spec. ES4 (endeavori), Pyrococcus spec. OT3 (horikoshii), Pyrococcus profundus, Thermococcus stetteri, Thermococcus Spec. AN1 (zilligii), Thermococcus peptonophilus, Thermococcus celer, Thermococcus fumicolans, Pyrodictium occultum, Pyrodictium abyssi oder Thermosipho africanus.

Beispiele für geeignete reverse Transkriptasen sind MMLV reverse Transkriptase, AMV reverse Transkriptase, RSV , HIV-1 reverse Transkriptase und HIV-2 reverse Transkriptase, ohne auf diese beschränkt zu sein.

In einer bevorzugten Ausführungsform ist das Enzym, das gemäß der Erfindung durch eine chemische Modifikation reversibel inhibiert ist wenigstens eine dieser thermostabilen Polymerasen, die durch reversible Verknüpfung wenigstens eines ihrer Lysinreste mit einem Citraconsäureanhydrid reversibel inhibiert ist, wobei die Inhibierung durch Wärmezufuhr rückgängig gemacht werden kann (Details dieser reversibeln Verknüpfung siehe US Patente 5,773,258 und 5,677,152), oder durch reversible Verknüpfung bzw. Vernetzung der Aminosäureseitenketten mit einem Aldehyd, bevorzugt einem Formaldehyd, wobei auch hier die dadurch entstehende Inhibierung durch Wärmezufuhr rückgängig gemacht werden kann (Details dieser Verknüpfung siehe US Patent 6,182,998). Die Polymerase, die durch nicht-kovalente Bindung eines Polyanions reversibel inhibiert ist, ist gemäß der bevorzugten Ausführungsform der Erfindung ebenfalls wenigstens eine der oben genannten Polymerasen, welche jedoch bei Raumtemperatur durch ein Polyanion komplexiert ist, wie es z.B. in US 6,667,165 beschrieben ist.

Bevorzugt werden die Enzyme entsprechend der vorliegenden Erfindung in einem Unit-Verhältnis von chemisch modifiziertem Enzym zu durch nicht-kovalente Bindung modifiziertem Enzym von 0,1:1 bis 100:1 eingesetzt.
Die Unit-Definition der verschiedenen, hier erwähnten Enzyme lautet wie folgt:
DNA-Polymerasen: Eine Unit ist definiert als die Menge an Enzym, die unter optimierten Reaktionsbedingungen (Temperatur, Pufferzusammensetzung) in 30 Minuten 10 nmol dNTP in säure-unlösliches Material unter Verwendung von aktivierter DNA als Template einbaut.

Reverse Transkriptasen: Eine Unit ist definiert als die Menge an Enzym, die unter optimierten Reaktionsbedingungen (Temperatur, Pufferzusammensetzung) in 10 Minuten 1 nmol dNTP in säure-unlösliches Material unter Verwendung von poly(rA)·p(dT)12-18 als Template/Primer einbaut.

DNA-Ligasen: Eine Weiss-Unit ist definiert als die Menge an Enzym, die unter optimierten Reaktionsbedingungen (Temperatur, Pufferzusammensetzung) in 20 Minuten die Konversion von einem Nanomol [32PPi] in eine Norit-adsorbierbare Form katalysiert.

RNA-Ligasen.Eine Unit ist definiert als die Menge an Enzym, die unter optimierten Reaktionsbedingungen (Temperatur, Pufferzusammensetzung) in 30 Minuten die Umwandlung von 1 nmol an 5'-Phosphoryl-Enden in 5'-[32P]-poly(A)12-18 in eine Phosphatase-resistente Form katalysiert.

Restriktions-Endonucleasen: Eine Unit ist definiert als die Menge an Enzym, die unter optimierten Reaktionsbedingungen (Temperatur, Pufferzusammensetzung) in einer Stunde den kompletten Verdau von 1 µg an Substrat-DNA katalysiert.

Exonucleasen: Eine Unit ist definiert als die Menge an Enzym, die unter optimierten Reaktionsbedingungen (Temperatur, Pufferzusammensetzung) in 30 Minten die Freisetzung von 1 nmol säurelöslicher Nukleotide katalysiert.

In einer bevorzugten Ausführungsform der Erfindung werden die beiden Enzyme dazu verwendet RNA- oder DNA- Proben gemäß bekannter gentechnischer Verfahren zu bearbeiten oder/und zu vervielfältigen. Solche Verfahren sind beispielsweise die reverse Transkription von RNA zu cDNA, die gekoppelte Reverse Transkrition und PCR Amplifikation in einer Reaktion ("one-step RT-PCR"), die Amplifikation von DNA mit Hilfe einer Polymerase-Kettenreaktion (PCR), das Schneiden von DNA mit Hilfe von Restriktionsenzymen, das Ligieren von DNA und ähnliche Verfahren, ohne auf diese beschränkt zu sein. Ein besonders bevorzugtes "Bearbeitungsverfahren" ist die PCR, ggf. mit einer vorgeschalteten reversen Transkription, die zu einer Vervielfältigung (Amplifikation) spezifischer DNA-Sequenzen führt.

Das Vorgehen bei einer PCR, ggf. mit vorgeschalteter reverser Transkription von RNA zu cDNA, ist dem Fachmann bekannt und soll im Folgenden anhand eines Beispiels nur kurz umrissen werden. Es ist hier festzuhalten, dass es neben der unten beispielhaft aufgeführten PCR mit einem sequenzspezifischen Primerpaar verschiedene Variationsmöglichkeiten der PCR gibt, z.B. durch Einsetzen von Primern, die absichtlich "mismatches" enthalten, oder durch verschiedene Kombinationen von passenden mit weniger passenden Primern, wobei alle diese Varianten mit dem hier beschriebenen erfindungsgemäßen Ansatz durchzuführen sind.

Bei der reversen Transkription wird aus RNA (einzelsträngig), bevorzugt mRNA, zunächst ein RNA-DNA-Hybrid erstellt. Die so hergestellte DNA-Matritze (cDNA) entspricht (komplementär) der auf der mRNA "abgelegten" Information.

Durch Einsetzen solcher cDNA oder auch einer anderweitig isolierten DNA-Probe in eine PCR kann unter Anwendung sequenzspezifischer Primer doppelsträngige DNA hergestellt werden, wobei optimalerweise nur die Sequenzen vervielfältigt werden, die die Sequenzen der Primer umfassen. In jedem Zyklus der PCR wird der DNA-Doppelstrang zunächst geschmolzen (die Stränge werden getrennt), dann erfolgt eine Anlagerung zueinander passender Sequenzen, bevorzugt der Primer an ihr Template, gefolgt vom Auffüllen der einzelsträngigen Bereiche durch "Anknüpfen" der jeweils "passenden" Nucleotide durch die DNA-Polymerase, wodurch wieder ein DNA-Doppelstrang erhalten wird. Da in jedem Zyklus die im vorherigen Zyklus erstellten DNA-Sequenzen zusätzlich als Template zur Verfügung stehen, ergibt sich eine exponentielle Zunahme der DNA-Sequenz(en), die die Primersequenzen umfassen. Aus diesem Grund wird in einer PCR mit zunehmender Zyklenzahl immer mehr Polymeraseaktivität erforderlich, um die Vervielfältigung der spezifischen Sequenz auch tatsächlich zu gewährleisten.

Insbesondere in diagnostischen Ansätzen oder bei der sogenannten "real time PCR" ist es wichtig, dass eine ausreichende Enzymaktivität zu jedem Zeitpunkt gewährleistet ist, so dass es weder zu einem zu frühen "Ausscheren" aus der exponentiellen Zunahme der gewünschten PCR kommt, noch zu Kettenabbrüchen, weil z.B. der nächste Zyklus (beginnend mit Schmelzen der doppelsträngigen DNA) beginnt, bevor die Polymerase den Einzelstrang vollständig auffüllen konnte. Beides führt zu einer Verschlechterung der Sensitivität des Verfahrens.

Die vorliegende Erfindung beruht auf der Erkenntnis, dass beim alleinigen Einsatz von Polymerasen, die durch eine nicht-kovalente Bindung eines Inhibitors reversibel inhibiert sind insbesondere bei kleinen Template-Ausgangsmengen oder einer geringen Kopienzahl des Zielmoleküls (DNA mit gewünschter Sequenz) zu Beginn einer PCR durch nahezu vollständige Aktivierung des Enzyms unspezifische Primer-Hybridisierungsereignisse zur Amplifikation unspezifischer Produkte führt. Sofern die Zusammensetzung des verwendeten Reaktionspuffers keine hohe Hybridisierungsspezifität gewährleistet und/oder die Sequenzen der Primer ein wechselseitiges Hybridisieren begünstigen, besteht aufgrund der hohen Polymeraseaktivität von Beginn der PCR an ein erhöhtes Risiko der Bildung von unspezifischen PCR-Produkten. Die am Anfang der PCR gebildeten unspezifischen "Nebenprodukte" dienen in den folgenden Zyklen ebenso als Template, wie die an sich gewünschte Sequenz.

Beim alleinigen Einsatz von chemisch modifizierten Enzymen besteht dagegen die oben beschriebene Problematik, dass ggf. zu Beginn der PCR keine ausreichende Polymeraseaktivität zur Verfügung steht, um im Laufe des PCR-Zyklus die DNA-Sequenz, an die die Primer spezifisch gebunden haben jeweils vollständig aufzufüllen, so dass es hier zu unerwünschten Kettenabbrüchen kommen kann. Von den Herstellern solcher durch chemische Modifikation inhibierter Enzyme wird daher empfohlen eine initiale Inkubation der Proben über einen längeren Zeitraum hinweg (typischerweise 2 bis 15 Min) bei Temperaturen über 90°C durchzuführen. Dennoch haben diese Enzyme auch nach einer solchen Inkubationszeit üblicherweise noch nicht ihre vollständige Aktivität wiedererlangt. Erst nach mehreren Inkubationszyklen mit Temperaturen in diesem Bereich kann von einer bestmöglichen Aktivierung ausgegangen werden.

Aus dieser Erkenntnis wurde gemäß der vorliegenden Erfindung der Ansatz gewählt für die Bearbeitung und Vervielfältigung von DNA-Sequenzen, insbesondere in einer PCR sowohl eine "schnell aktivierbare" Polymerase, als auch ein über die Zeit "zunehmend aktivierbares" Enzym einzusetzen, um optimale Ergebnisse zu erhalten und die oben beschriebenen Nachteile zu minimieren.

In eine Zusammensetzung zur Bearbeitung von DNA, z.B. einen PCR-Ansatz, wird somit beispielsweise sowohl eine durch nicht-kovalente Bindung eines Polyanions reversibel inhibierte thermostabile DNA-Polymerase in einer Menge eingesetzt, die ausreicht zu Beginn der PCR eine DNA-Polymerase-Aktivität bereitzustellen, die nach einer spezifischen Hybridisierung eines Primers an die einzelsträngige DNA das vollständige "Auffüllen" zum Doppelstrang gewährleistet, als auch eine durch chemische Modifikation reversibel inhibierte thermostabile DNA-Polymerase in einer Menge, die gewährleistet, dass im Laufe der Zyklen der PCR eine ausreichende Enzymaktivität bereitsteht, so dass die exponentielle Vervielfältigung der DNA auch noch in "späten" Zyklen gewährleistet ist. Somit wird gemäß diesem Ansatz in jeder Phase der PCR die erforderliche Menge an aktivem Enzym den zu amplifizierenden Templatemengen angepasst.

Bevorzugt wird dieser Ansatz dann gewählt, wenn die Größe des zu amplifizierenden Amplicons größer als ungefähr 200 bp ist, da insbesondere bei größeren Amplicons die Gefahr des Kettenabbruchs durch unvollständige Synthese des komplementären Stranges besteht, wenn keine ausreichende Enzymaktivität im Ansatz vorliegt.

In einer bevorzugten Ausführungsform wird die "schnell aktivierbare", also durch nicht-kovalente Bindung eines Polyanions reversibel inhibierte Polymerase zu der "zunehmend aktivierbaren", also durch chemische Modifikation reversibel inhibierten DNA-Polymerase in einem Unit-Verhältnis von 1:0,1 bis 1:100 eingesetzt. Besonders bevorzugt wird die schnell aktivierbare Polymerase maximal in denselben Anteilen, wie die chemisch modifizierbare Polymerase eingesetzt, weiter bevorzugt im Unterschuß gegenüber der chemisch modifizierten Polymerase.

Gemäß der Erfindung enthält eine Zusammensetzung oder ein Kit, bevorzugt eine Zusammensetzung oder ein Kit für die Bearbeitung oder Vervielfältigung von Polynucleotiden, insbesondere bevorzugt für die Durchführung einer PCR, beide Enzyme nebeneinander. Darüber hinaus kann die Zusammensetzung oder der Kit je nach gewünschter Anwendung weitere benötigte Materialien oder Reagenzien enthalten, die für die gewünschte Anwendung geeignet oder nötig sind. So kann beispielsweise eine Zusammensetzung oder ein Kit für eine PCR neben den Enzymen einen geeigneten Puffer, dNTPs (dATP, dITP, dUTP, dGTP, dTTP dCTP oder Derivate davon), ggf. Cofaktoren wie z.B. MgCl₂, ggf. Primer, ggf. DNA als Referenztemplate, oder weitere Komponenten enthalten. Die für eine reverse Transkription, eine Ligation oder ein Schneiden mit Hilfe von Restriktionsnucleasen benötigten oder geeigneten Komponenten sind dem Fachmann vollständig bekannt und können jedem Laborhandbuch oder der Fachliteratur entnommen werden.

### Abbildungen:

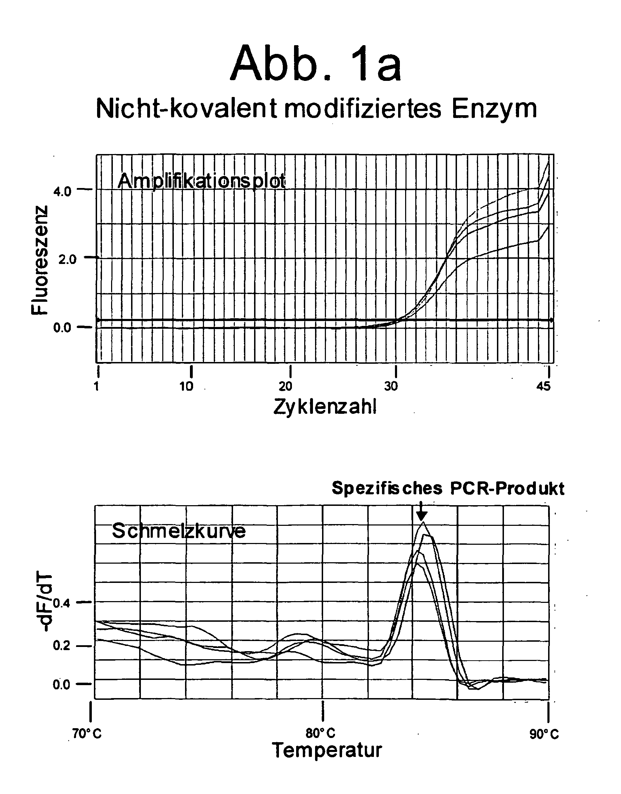

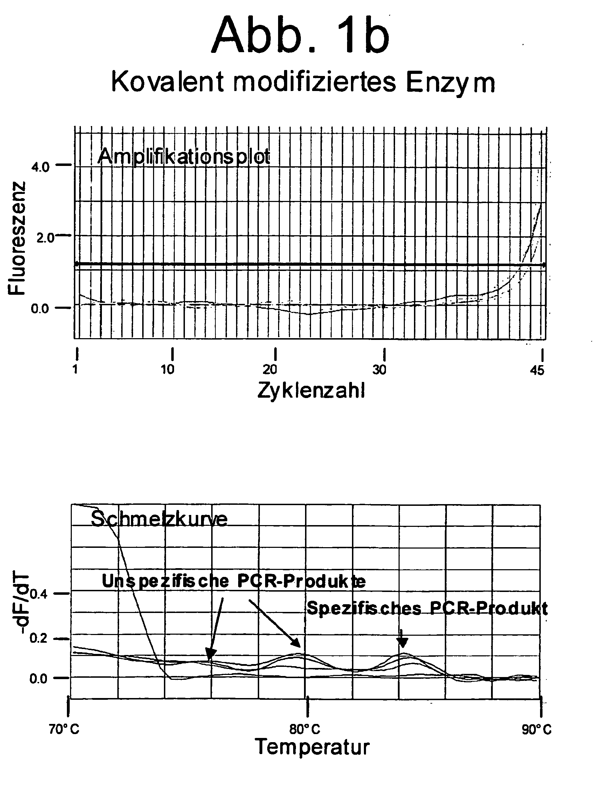

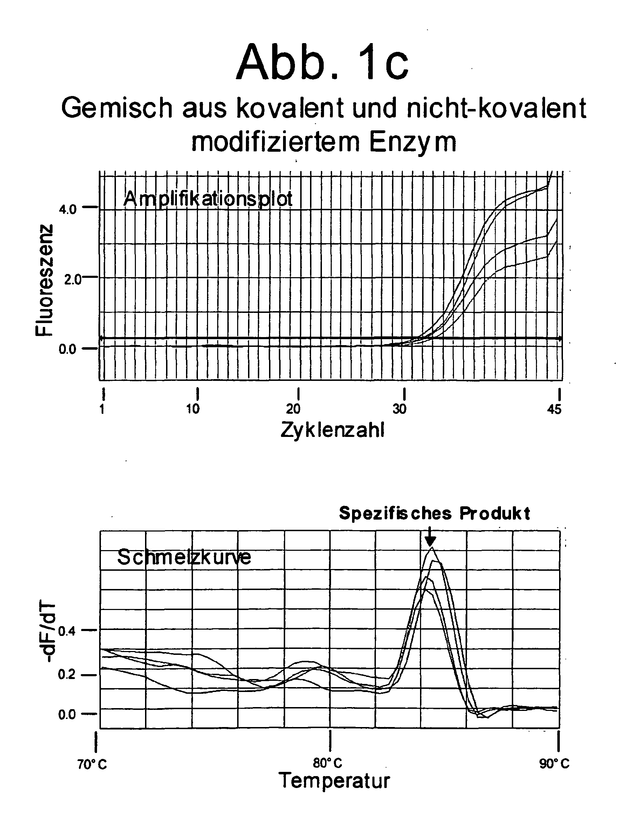

Abbildung 1 a zeigt im oberen Teil den Amplifikationsplot , der die Zunahme doppelsträngiger DNA im Verlauf einer PCR durch Amplifikation mit nicht-kovalent modifiziertem Enzym zeigt. Details der PCR sind Beispiel 1 a zu entnehmen. Im unteren Teil zeigt Abbildung 1a die Schmelzkurve des so erhaltenen PCR Produkts.
Abbildung 1b zeigt im oberen Teil den Amplifikationsplot , der die Zunahme doppelsträngiger DNA im Verlauf einer PCR durch Amplifikation mit kovalent modifiziertem Enzym zeigt. Details der PCR sind Beispiel 1 b zu entnehmen. Im unteren Teil zeigt Abbildung 1b die Schmelzkurve des/der so erhaltenen PCR Produkte(s).
Abbildung 1c zeigt im oberen Teil den Amplifikationsplot , der die Zunahme doppelsträngiger DNA im Verlauf einer PCR durch Amplifikation mit einem Gemisch aus nicht-kovalent modifiziertem Enzym und kovalent modifiziertem Enzym zeigt. Details der PCR sind Beispiel 1 c zu entnehmen. Im unteren Teil zeigt Abbildung 1c die Schmelzkurve des so erhaltenen PCR Produkts.

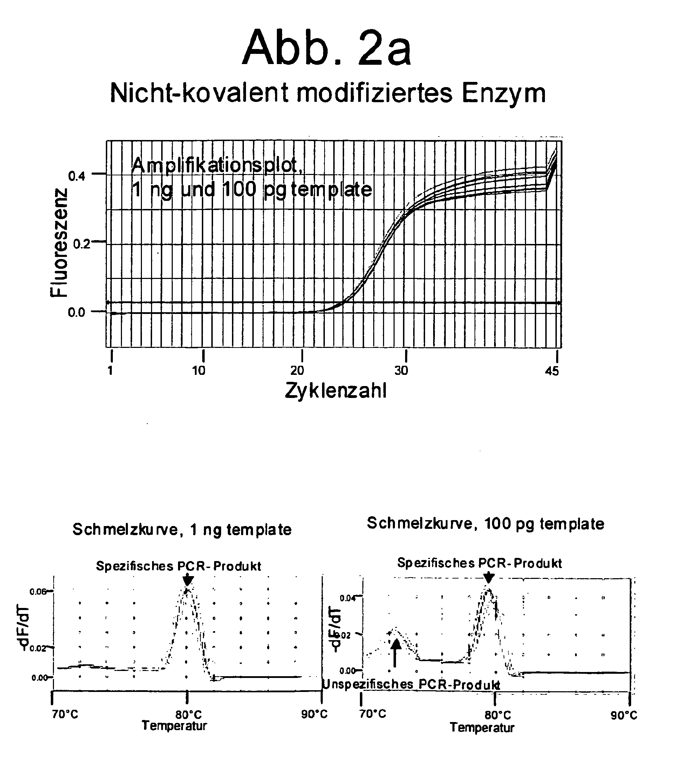

Abbildung 2a zeigt im oberen Teil den Amplifikationsplot, der die Zunahme doppelsträngiger DNA bei Einsatz verschiedener Ausgangsmengen an DNA im Verlauf einer PCR durch Amplifikation mit nicht-kovalent modifiziertem Enzym zeigt. Details der PCR sind Beispiel 2a zu entnehmen. Im unteren Teil zeigt Abbildung 2a die Schmelzkurven der so erhaltenen PCR Produkte.

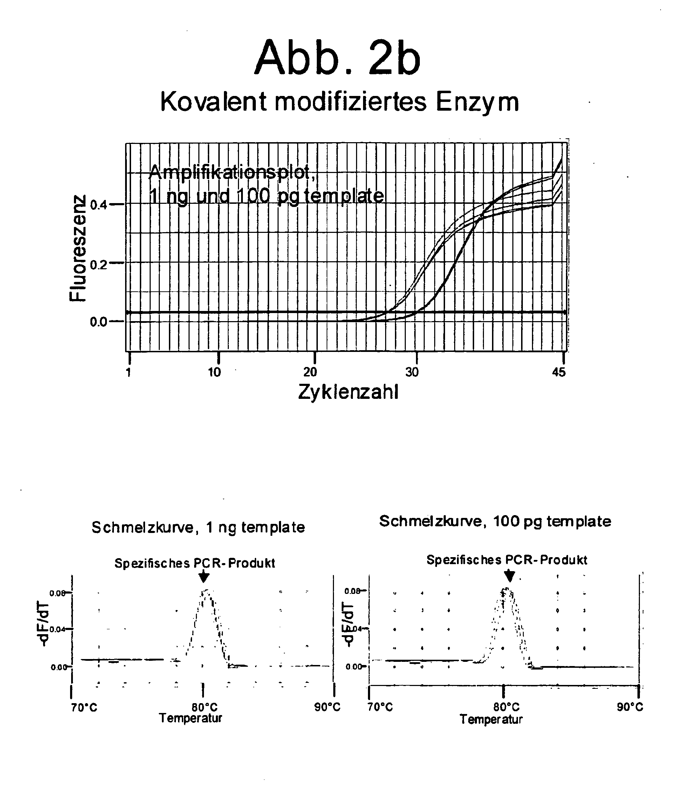

Abbildung 2b zeigt im oberen Teil den Amplifikationsplot, der die Zunahme doppelsträngiger DNA bei Einsatz verschiedener Ausgangsmengen an DNA im Verlauf einer PCR durch Amplifikation mit kovalent modifiziertem Enzym zeigt. Details der PCR sind Beispiel 2b zu entnehmen. Im unteren Teil zeigt Abbildung 2b die Schmelzkurven der so erhaltenen PCR Produkte.

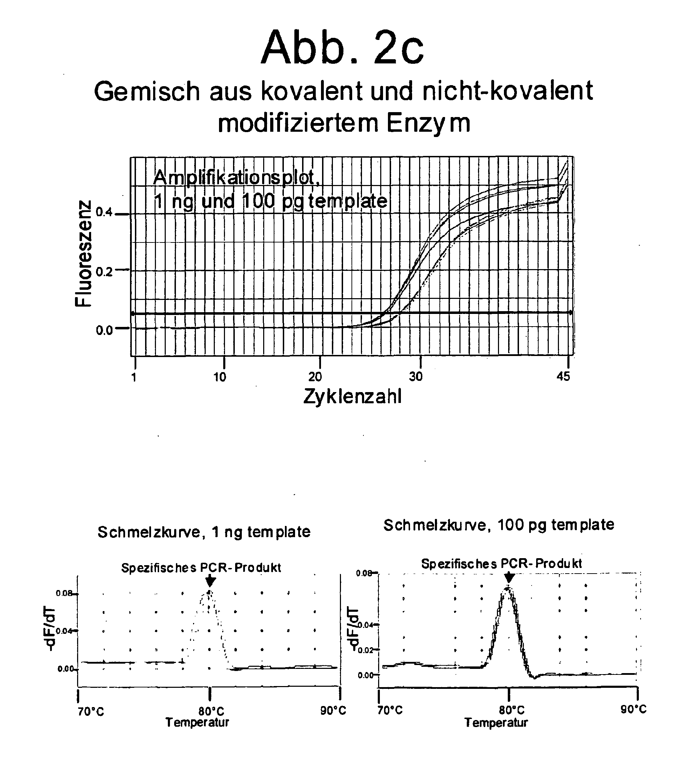

Abbildung 2c zeigt im oberen Teil den Amplifikationsplot , der die Zunahme doppelsträngiger DNA bei Einsatz verschiedener Ausgangsmengen an DNA im Verlauf einer PCR durch Amplifikation mit einem Gemisch aus nicht-kovalent modifiziertem Enzym und kovalent modifiziertem Enzym zeigt. Details der PCR sind Beispiel 2c zu entnehmen. Im unteren Teil zeigt Abbildung 2c die Schmelzkurve der so erhaltenen PCR Produkte.

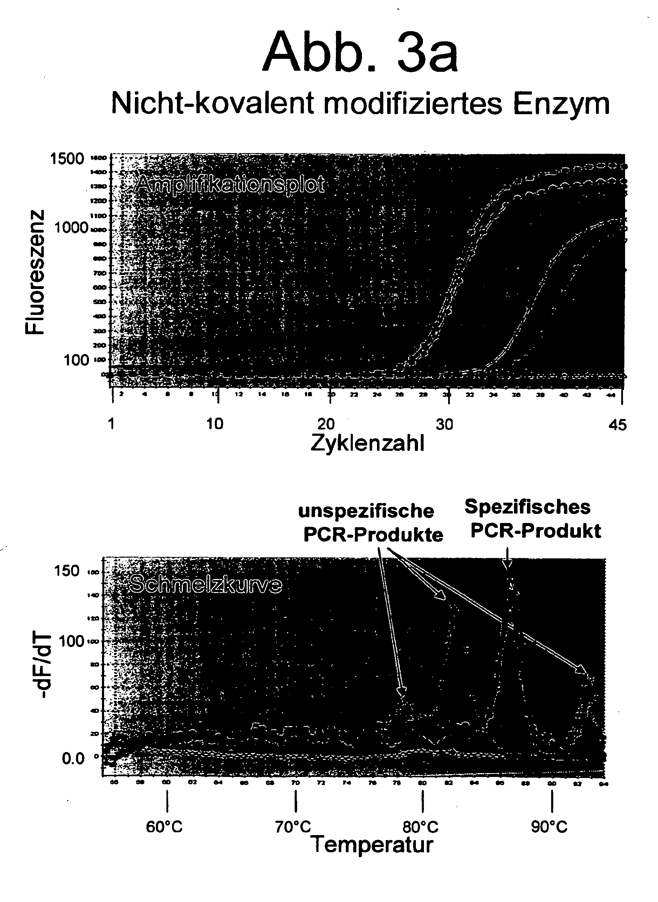

Abbildung 3a zeigt im oberen Teil den Amplifikationsplot, der die Zunahme doppelsträngiger DNA im Verlauf einer PCR durch Amplifikation mit nicht-kovalent modifiziertem Enzym zeigt. Details der PCR sind Beispiel 3a zu entnehmen. Im unteren Teil zeigt Abbildung 3a die Schmelzkurve der so erhaltenen PCR Produkte.

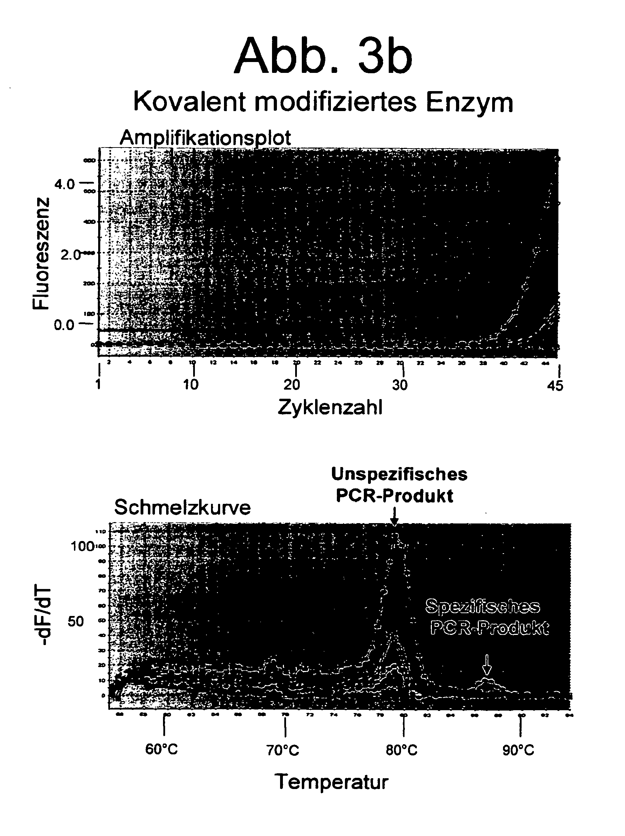

Abbildung 3b zeigt im oberen Teil den Amplifikationsplot , der die Zunahme doppelsträngiger DNA im Verlauf einer PCR durch Amplifikation mit kovalent modifiziertem Enzym zeigt. Details der PCR sind Beispiel 3b zu entnehmen. Im unteren Teil zeigt Abbildung 3b die Schmelzkurve der so erhaltenen PCR Produkte.

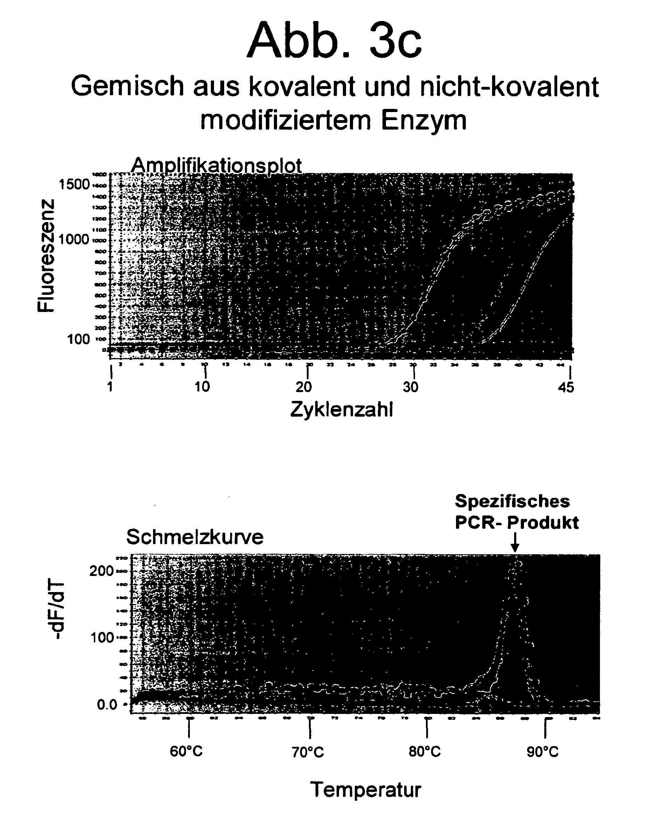

Abbildung 3c zeigt im oberen Teil den Amplifikationsplot, der die Zunahme doppelsträngiger DNA im Verlauf einer PCR durch Amplifikation mit einem Gemisch aus nicht-kovalent modifiziertem Enzym und kovalent modifiziertem Enzym zeigt. Details der PCR sind Beispiel 3c zu entnehmen. Im unteren Teil zeigt Abbildung 3c die Schmelzkurve des so erhaltenen PCR Produkts.

### Beispiele:

Die nachfolgenden Beispiele sind dazu gedacht die Erfindung weiter zu erläutern, ohne dass diese auf die Ausführungsbeispiele eingeschränkt sein soll. Beispiel 3 fällt nicht unter den Anspruchs Umfang der vorliegenden Erfindung.

### Beispiel 1:

### Amplifikation eines langen PCR Fragments in real-time PCR mit SYBR Green basierender Detektion mit kovalent und nicht-kovalent modizifierten DNA-Polymerasen

Um den Effekt unterschiedlich modifizierter thermostabiler DNA-Polymerasen auf die Amplifikation langer DNA-Fragmente zu untersuchen, wurde ein 690-Basenpaar Fragment des Transkripts für das humane CTNNA1-Gen (NM_001903) amplifiziert. Die hierfür verwendeten Primer hatten folgende Sequenzen:
Forward Primer: AGCTGAAAGTTGTGGAAGATG (Seq ID No:1)
Reverse Primer: GGAGCTGTCTACGCAAGTC (Seq ID No:2)
Die Amplifikation wurde durchgeführt mit einer abgewandelten Version des QuantiTect SYBR Green PCR Master-Mix (QIAGEN GmbH, Hilden), der mit Außnahme von Primern und Template alle erforderlichen Komponenten für die SYBR Greenbasierende real-time PCR enthält (d.h. Tris, Kaliumchlorid, Magnesiumchlorid, Ammoniumsulfat, dNTP-Nucleotide, SYBR Green). Die Abwandlung bestand im Herauslassen der HotStarTaq DNA Polymerase (QIAGEN), die im kommerziell erhältlichen Produkt bereits enthalten ist. Stattdessen wurden jeweils folgende Enzyme eingesetzt:
1. HotMasterTaq DNA-Polymerase (Eppendorf AG, Hamburg), ein Enzym, das gemäß US Patent 6,667,165 durch nicht-kovalente Komplexierung mit einem nicht-nukleinsäureartigen Polyanion temperaturabhängig inhibiert wird und bei Temperaturen über 55°C seine volle Aktivität entfaltet (im folgenden "nicht-kovalent modifiziertes Enzym" genannt): Die eingesetzte Menge betrug 1.5 Units pro Reaktion
2. Taq-DNA Polymerase, die gemäß US Patent 6,183,998 mit Formaldehyd kovalent modifiziert und damit inhibiert war (Qiagen, Hilden) (im folgenden "kovalent modifiziertes Enzym" genannt). Die eingesetzte Menge betrug 1.25 Units pro Reaktion
3. Ein Gemisch aus dem nicht-kovalent sowie dem kovalent modifizierten Enzym, im Verhältnis 0.125 Units nicht-kovalent modifiziertes Enzym zu 1.25 Units kovalent modifziertes Enzym pro Reaktion.

Das finale Volumen der Reaktion betrug jeweils 25 µl, die Primer wurden in einer finalen Konzentration von 0,3 µM eingesetzt. Die Durchführung erfolgte auf dem ABI PRISM 7000 Sequence Detection System (Applied Biosystems). Als Template diente 1 ng cDNA, die aus HeLa-RNA hergestellt worden war, pro Reaktion. Pro Enzym bzw. Enzym-Mischung wurden jeweils 4 Replikate analysiert.

Die Reaktionsbedingungen waren wie folgt:
A: Initiale Denaturierung des Templates / Aktivierung des Enzyms 95°C, 5 Minuten
B: Amplifikation:
   Denaturierung: 95°C, 15 Sekunden
   Primer-Annealing: 55°C, 30 Sekunden
   Primer-Extension: 72°C, 30 Sekunden
   Fluoreszenzaufnahme während der Extension in jedem Zyklus Anzahl der Zyklen: 45
C: Schmelzkurvenanalyse
   Initiale Denaturierung des PCR-Produkts: 95°C, 15 Sekunden
   Rehybridisierung des PCR-Produkts: 60°C, 20 Sekunden
   Aufschmelzen des PCR-Produkts: Aufheizen von 60°C auf 95 Grad mit einer Heizgeschwindigkeit von ca. 0.2 °C/sec
   Fluoreszenzaufnahme während des Aufschmelzens des PCR Produkts

Der Ansatz der Reaktionen erfolgte bei Raumtemperatur.

Wie aus Abbildung 1a deutlich hervorgeht, gelang mit Hilfe des nicht-kovalent modifizierten Enzyms die Amplifikation des gewünschten 690bp-Fragments, was sich aus der kombinierten Analyse des Amplifikations-Plots sowie der Schmelzkurvenanalyse ergibt. Der "Threshold", das heißt, der Fluoreszenz-Wert, der deutlich oberhalb der Hintergrundfluoreszenz liegt, wird bei PCR-Zyklus 30.5 erreicht. Die Schmelztemperatur des PCR-Produkts liegt bei den erwarteten 84°C; der Anteil an weiteren, unspezifischen Produkten ist im Vergleich zum spezifischen Produkt vernachlässigbar. Für ein derartiges, langes Amplifikat ist also ein sofort reaktivierbares, nicht-kovalent modifiziertes Enzym vorteilhaft.

Abbildung 1b belegt den Misserfolg der Amplifikation desselben Fragments bei alleiniger Verwendung einer kovalent modifizierten thermostabilen DNA-Polymerase. Zum einen erfolgt erst in einem sehr späten PCR-Zyklus (43,5) ein erkennbarer Anstieg der Fluoreszenz, zudem nur für drei von vier Replikaten. Zum anderen ergibt sich aus der Schmelzkurvenanalyse, dass es sich bei einem Großteil der gebildeten Produkte um unerwünschte Amplifikate wie Primer-Dimere und weitere unspezifische Reaktionsprodukte handelt.

Die hier gewählte Methode der Detektion mit Hilfe des Fluoreszenz-Farbstoffs SYBR Green diskriminiert nicht zwischen spezifischem und unspezifischem PCR Produkt. SYBR Green ist ein Farbstoff, der sich sequenzunabhängig an doppelsträngige DNA anlagert und nach erfolgter Anlagerung und gleichzeitiger Excitation mittels einer Lichtquelle Fluoreszenz emittiert. Das bedeutet für das in Abbildung 1 b gezeigte Beispiel, dass der Anstieg der Fluoreszenz im Amplifikationsplot nicht nur sehr spät erfolgt, sondern auch noch primär auf der Detektion unspezifischer Produkte beruht. Für ein derartiges, langes Amplifikat ist also ein nicht sofort vollständig reaktivierbares, kovalent modifiziertes Enzym nicht vorteilhaft

Die Abbildung 1c belegt die vorteilhafte Kombination einer Kombination von kovalent und nicht-kovalent modifizierten Enzymen. Durch die Beimengung einer kleinen Menge an sofort reaktivierbarem, nicht-kovalent modifiziertem Enzym gelang es, die Resultate, wie sie in Abbildung 1b gezeigt wurden, drastisch zu verbessern und Ergebnisse zu erzielen, die den unter Abbildung 1a gezeigten sowohl bezüglich Sensitivität (vergleichbare Zyklenzahl beim Kreuzen des Thresholds) als auch Spezifität (hohe Ausbeute an spezifischem Produkt) sehr ähneln.

### Beispiel 2:

### Amplifikation eines PCR Fragments in real-time PCR mit SYBR Green basierender Detektion mit kovalent und nicht-kovalent modizifierten DNA-Polymerasen unter Verwendung einer zur Dimer-Bildung neigenden Primer-Kombination

Um den Effekt unterschiedlich modifizierter thermostabiler DNA-Polymerasen auf die Amplifikation unter Verwendung von zur Dimer-Bildung neigenden Primern zu untersuchen, wurden ein 129-Basenpaar Fragment des Transkripts für das humane MYC-Gen (NM_002467) amplifiziert. Die hierfür verwendeten Primer hatten folgende Sequenzen:
Forward Primer: TGCTCCATGAGGAGACA (Seq ID No:3)
Reverse Primer: GTGATCCAGACTCTGACCTTT (Seq ID No:4)

Das Ansetzen der Reaktionen erfolgte wie unter Beispiel 1 beschrieben. Abweichend von Beispiel 1 wurden in einem Ansatz 1 ng, in einem anderen Ansatz 10pg cDNA, die aus HeLa-RNA hergestellt worden war, als Template-Menge pro Reaktion eingesetzt. Die Durchführung erfolgte diesmal auf dem ABI PRISM 7500 Sequence Detection System (Applied Biosystems).

Wie aus Abbildung 2a deutlich hervorgeht, gelang es mit Hilfe des nicht-kovalent modifizierten Enzyms nicht, die beiden unterschiedlichen Template-Mengen zu diskriminieren. Die Analyse des Amplifikationsplots zeigt, dass alle Proben den Fluoreszenz-Schwellenwert beim selben PCR-Zyklus kreuzen. Dabei ist zu erwarten, dass bei Verwendung der geringeren Template-Menge ein deutlich späteres Überschreiten des Schwellenwerts auftritt. Die Ursache hierfür ergibt sich aus der getrennten Analyse der Schmelzkurve für die beiden verwendeten Template-Mengen. Während es bei der Verwendung der höheren Template-Menge zur ausschließlichen Bildung des spezifischen PCR-Produkts mit einer Schmelztemperatur von 80°C kommt, bilden sich bei der kleineren Template-Menge neben dem spezifischen Produkt auch noch unspezifische PCR-Produkte, die anhand ihrer geringeren Schmelztemperatur als typische Primer-Dimere identifiziert werden können. Das Entstehen dieser Primer-Dimere ist auf die hohe Anfangsaktivität der DNA-Polymerase unmittelbar nach der initialen Denaturierung zurückzuführen. Da bei geringer Template-Menge der Anteil an nicht-annealten Primern besonders hoch ist, führt eine vergleichsweise hohe Menge an aktiver Polymerase von Beginn der Reaktion an zu einer verstärkten Formation derartiger PCR-Artefakte. Da diese, wie unter Beispiel 1 erläutert, gleichfalls zur Generierung des Fluoreszenz-Signals beitragen, ist eine Diskriminierung zwischen den beiden Template-Mengen und damit die gewünschte Quantifizierung, nicht mehr möglich. Für ein derartiges Amplifikat mit zur Dimer-Bildung neigenden Primern ist also ein sofort reaktivierbares, nicht-kovalent modifiziertes Enzym nicht vorteilhaft.

Abbildung 2b belegt die erfolgreiche spezifische Amplifikation desselben Fragments bei alleiniger Verwendung einer kovalent modifizierten thermostabilen DNA-Polymerase und somit eine gelungene Diskriminierung der beiden verwendeten Template-Mengen. Im Amplifikationsplot zeigt sich das erwartete, Zyklus-versetzte Kreuzen des Fluoreszenz-Thresholds; die Schmelzkurve belegt die spezifische Amplifikation für beiden Template Mengen.. Für ein derartiges Amplifikat mit zur Dimer-Bildung neigenden Primern ist also ein nicht sofort vollständig reaktivierbares, kovalent modifiziertes Enzym vorteilhaft.

Die Abbildung 2c belegt die vorteilhafte Kombination von kovalent und nichtkovalent modifizierten Enzymen. Durch die Beimengung einer kleinen Menge an sofort reaktivierbarem, nicht-kovalent modifiziertem Enzym gelang es, die Resultate, wie sie in Abbildung 2a gezeigt wurden, drastisch zu verbessern und Ergebnisse zu erzielen, die den unter Abbildung 2b gezeigten sowohl bezüglich Sensitivität (vergleichbare Zyklenzahl beim Kreuzen des Thresholds) als auch Spezifität (hohe Ausbeute an spezifischem Produkt) sehr ähneln.

### Beispiel 3:

### Amplifikation eines PCR Fragments in real-time PCR mit auf SYBR Green basierender Detektion mit kovalent und nicht-kovalent modizifierten DNA-Polymerasen, die unter Verwendung unterschiedlicher Methoden der kovalenten bzw. nichtkovalenten Modifikation produziert wurden

Um zu belegen, dass der vorteilhafte Effekt der Kombination unterschiedlich modifizierter thermostabiler DNA-Polymerasen nicht auf eine bestimmte Art der kovalanten und nicht-kovalenten Modifizierung beschränkt ist, wurde für die in diesem Beispiel aufgeführte PCR eine Kombination von zwei Enzymen eingesetzt, die sich beide in ihrer Art der Modifikation von den in Beispiel 1 und 2 eingesetzten Enyzmen unterscheiden. Es handelte sich um die folgenden beiden DNA Polymerasen.

### 1: Kovalent-modifiziertes Enzym:

AmpliTaq Gold (Applied Biosystems,Foster City, CA, USA), eine kovalent modifizierte Version der Taq DNA Polymerase, die durch Behandlung mit einem Säureanhydrid reversibel inhibiert ist (Details sind den US Patenten 5,677,152 und 5,773,258 zu entnehmen)

### 2: Nicht-kovalent modifziertes Enzym:

Platinum® Taq DNA Polymerase (Invitrogen, Carlsbad, CA, USA), eine rekombinante Taq DNA Polymerase, die mit einem Antikörper komplexiert ist, der die Polymerase-Aktivität bei Raumtemperatur blockiert. Durch Erwärmung auf 94°C, wie dies typischerweise während des Denaturierungs-Schritts geschieht, wird der Antikörper denaturiert und die Blockierung aufgehoben (Details sind den US Patenten 5,338,671 und 5,587,287 zu entnehmen).

Um diese bezüglich der Amplifikation langer DNA-Fragmente zu untersuchen, wurde ein 112-Basenpaar Fragment des Transkripts für das humane AK1-Gen (NM_000476) amplifiziert. Die hierfür verwendeten Primer hatten folgende Sequenzen:
Forward Primer: AAGAGAAGCTGAAGAAAACCAA (Seq ID No:5)
Reverse Primer: GTGGAGAGGTGGGTGTAG (Seq ID No:6)
Die Amplifikation wurde durchgeführt unter Verwendung des HotStarTaq PCR Puffers, der Tris, Kaliumchlorid, Magnesiumchlorid und Ammoniumsulfat enthält. Darüber hinaus wurde SYBR Green in einer finalen Konzentration von 0.1 x sowie dNTP-Nukleotide in einer finalen Konzentration von 0.2 mM zugegeben. Die oben angeführten Enzyme wurden folgendermaßen eingesetzt.
4. AmpliTaq Gold DNA Polymerase alleine: Die eingesetzte Menge betrug 1.25 Units pro Reaktion (gemäß Empfehlung des Herstellers)
5. Platinum Taq DNA Polymerase alleine: Die eingesetzte Menge betrug 0.5 U (gemäß Empfehlung des Herstellers)
6. Ein Gemisch aus AmpliTaq Gold DNA Polymerase-(kovalent modifiziertes Enzym) sowie Platinum Taq DNA Polymerase (nicht-kovalent modifiziertes Enzym), im Verhältnis 0.125 Units nicht-kovalent modifiziertes Enzym zu 1.25 Units kovalent modifziertes Enzym pro Reaktion.

Das finale Volumen der Reaktion betrug jeweils 25 µl, die Primer wurden in einer finalen Konzentration von 0,7 µM eingesetzt. Die Durchführung erfolgte auf dem Mx3005P Real-Time PCR System (Stratagene). Als Template pro Reaktion dienten 10 ng, 1 ng und 0.1 ng cDNA, die aus HeLa-RNA hergestellt worden war. Pro Enzym bzw. Enzym-Mischung und pro Template-Menge wurden jeweils 4 Replikate analysiert.

Die Reaktionsbedingungen waren wie folgt:
A: Initiale Denaturierung des Templates / Aktivierung des Enzyms 95°C, 10 Minuten
B: Amplifikation:
   Denaturierung: 95°C, 15 Sekunden
   Primer-Annealing: 55°C, 30 Sekunden
   Primer-Extension: 72°C, 30 Sekunden
   Fluoreszenzaufnahme während der Extension in jedem Zyklus Anzahl der Zyklen: 45
C: Schmelzkurvenanalyse
   Initiale Denaturierung des PCR-Produkts: 95°C, 15 Sekunden
   Rehybridisierung des PCR-Produkts: 60°C, 20 Sekunden
   Aufschmelzen des PCR-Produkts: Aufheizen von 60°C auf 95 Grad mit einer Heizgeschwindigkeit von 0.1 °C/sec
   Fluoreszenzaufnahme während des Aufschmelzens des PCR Produkts

Der Ansatz der Reaktionen erfolgte bei Raumtemperatur.

Wie aus Abbildung 3a hervorgeht, gelang zwar mit Hilfe des nicht-kovalent modifizierten Enzyms die Amplifikation des gewünschten 112bp-Fragments, was sich aus der kombinierten Analyse des Amplifikations-Plots sowie der Schmelzkurvenanalyse ergibt. Es fällt allerdings auf, dass neben dem gewünschten Produkt mit einer Schmelztemperatur von ca. 88°C noch zahlreiche weitere Nebenprodukte, sowohl mit höherer als auch mit geringerer Schmelztemperatur auftreten. Eine zuverlässige Quantifizierung ist also nur bedingt möglich.

Abbildung 3b belegt den Misserfolg der Amplifikation desselben Fragments bei alleiniger Verwendung der kovalent modifizierten thermostabilen DNA-Polymerase. Der Anstieg der Fluoreszenz erfolgt erst zu einem sehr späten Zeitpunkt (ab Zyklus 39 für die höchste Template-Menge) bei vier Replikaten. Des weiteren belegt die Schmelzkurvenanalyse, dass es sich bei einem Großteil der gebildeten Produkte um unerwünschte, d.h. unspezifische Amplifikate handelt. Deren im Vergleich zum spezifischen Produkt geringerer Schmelzpunkt legt nahe, dass hier primär Primer-Dimere gebildet werden.

Die Abbildung 3c belegt die vorteilhafte Kombination einer Kombination von kovalent und nicht-kovalent modifizierten Enzymen, unabhängig von der Art der Modifizierung. Durch die Beimengung einer kleinen Menge an sofort reaktivierbarem, nicht-kovalent modifiziertem Enzym gelang es, die Resultate, wie sie in Abbildung 3a und 3b gezeigt wurden, drastisch zu verbessern und Ergebnisse zu erzielen, die sowohl bezüglich Sensitivität (Zyklenzahl beim Kreuzen des Thresholds) als auch Spezifität (Produktion von ausschließlich spezifischem Produkt) die Ergebnisse bei Verwendung beider Enyzme jeweils alleine deutlich übertreffen.

Die Kombination der Resultate aus den Beispielen 1 und 2 und 3 erlaubt den Rückschluß, dass eine Kombination von kovalent und nicht-kovalent modifizierten Enzymen ein universell einsetzbares Enyzmsystem darstellt, das den verschiedenen Ansprüchen einer PCR wie der effizienten Amplifikation langer Fragmente oder auch der spezifischen Amplfikation bei Verwendung kleiner Template-Mengen oder ungünstiger Primer-Kombinationen gerecht wird.

## Patentansprüche

1. Zusammensetzung, enthaltend sowohl mindestens ein durch chemische kovalente Modifikation reversibel inhibiertes Enzym, als auch mindestens eine durch nicht-kovalente Bindung eines Polyanions reversibel inhibierte Polymerase.

2. Kit, enthaltend mindestens ein durch chemische kovalente Modifikation reversibel inhibiertes Enzym und mindestens eine durch nicht-kovalente Bindung eines Polyanions reversibel inhibierte Polymerase, oder eine Mischung solcher Enzyme.

3. Verwendung eines Gemisches mindestens eines durch chemische kovalente Modifikation reversibel inhibierten Enzyms mit mindestens einer durch nichtkovalente Bindung eines Polyanions reversibel inhibierten Polymerase zur Bearbeitung oder Vervielfältigung von Polynucleotiden.

4. Verwendung gemäß Anspruch 3, wobei es sich bei der Bearbeitung oder Vervielfältigung um eine spezifische Amplifikation, eine Ligation, eine reverse Transkription oder ein Fragmentieren mittels Restriktionsschnitt(en) handelt.

5. Verwendung gemäß Anspruch 4, wobei die spezifische Amplifikation mittels PCR oder gekoppelter reverser Transkription-PCR (one-step RT-PCR) erfolgt.

6. Verfahren zur spezifischen Amplifikation von DNA oder RNA, wobei in wenigstens einem Reaktionsansatz sowohl mindestens ein durch chemische kovalente Modifikation reversibel inhibiertes Enzym, als auch mindestens eine durch nicht-kovalente Bindung eines Polyanions reversibel inhibierte Polymerase nebeneinander vorliegt.

7. Verfahren gemäß Anspruch 6 zur spezifischen Amplifikation von DNA oder RNA, wobei die spezifische Amplifikation mittels PCR oder gekoppelter reverser Transkription-PCR (one-step RT-PCR) erfolgt.

8. Zusammensetzung, Kit, Verwendung oder Verfahren gemäß einem der Ansprüche 1 bis 7, wobei es sich bei den Enzymen um thermostabile Enzyme handelt.

9. Zusammensetzung, Kit, Verwendung oder Verfahren gemäß einem der Ansprüche 1 bis 8, wobei die Inhibierung der Enzyme durch Zufuhr von Wärme rückgängig gemacht werden kann.

10. Zusammensetzung, Kit, Verwendung oder Verfahren gemäß einem der Ansprüche 1 bis 9, wobei es sich bei dem kovalent inhibierten Enzym um DNA-Polymerase, RNA-Polymerase, Ligase, reverse Transkriptase, Restriktions-Endonuklease oder Restriktions-Exonuklease und bei dem nichtkovalent inhibierten Enzym um DNA-Polymerase, RNA-Polymerase oder reverse Transkriptase handelt.

11. Zusammensetzung, Kit, Verwendung oder Verfahren gemäß Anspruch 10, wobei sowohl mindestens ein durch chemische kovalente Modifikation reversibel inhibiertes Enzym, als auch mindestens eine durch nicht-kovalente Bindung eines Polyanions reversibel inhibierte Polymerase eine thermostabile DNA-Polymerase ist.

12. Zusammensetzung, Kit, Verwendung oder Verfahren gemäß einem der Ansprüche 1 bis 11, wobei die Enzyme in einem Mischungsverhältnis von chemisch kovalent modifiziertem Enzym zu durch nicht-kovalente Bindung eines Polyanions modifizierter Polymerase in einem Unit-Mischungsverhältnis von 0,1:1 bis 100:1 vorliegen, bzw. eingesetzt werden.

13. Zusammensetzung oder Kit gemäß einem der Ansprüche 1, 2 oder 8 bis 12, zusätzlich enthaltend weitere Komponenten für eine PolymeraseKettenreaktion (PCR) oder gekoppelte Reverse Transkripton-PCR.

## Claims

1. A composition containing both at least one enzyme reversibly inhibited by chemical modification, and at least one polymerase reversibly inhibited by noncovalent binding of a polyanion.

2. A kit containing at least one enzyme reversibly inhibited by chemical modification and at least one polymerase reversibly inhibited by noncovalent binding of a polyanion, or a mixture of said enzymes.

3. Use of a mixture of at least one enzyme reversibly inhibited by chemical modification with at least one polymerase reversibly inhibited by noncovalent binding of a polyanion for the processing or amplification of polynucleotides.

4. The use as claimed in claim 3, wherein the processing or amplification is a specific amplification, a ligation, a reverse transcription or a fragmentation by restriction cut(s).

5. The use as claimed in claim 4, wherein the specific amplification takes place by PCR or coupled reverse transcription-PCR (one-step RT-PCR).

6. A method for specific amplification of DNA or RNA, wherein in at least one reaction charge, both at least one enzyme reversibly inhibited by chemical modification, and at least one polymerase reversibly inhibited by noncovalent binding of a polyanion are present together.

7. The method as claimed in claim 6 for the specific amplification of DNA or RNA, wherein the specific amplification takes place by PCR or coupled reverse transcription-PCR (one-step RT-PCR).

8. The composition, kit, use or method as claimed in one of claims 1 to 7, wherein the enzymes are thermostable enzymes.

9. The composition, kit, use or method as claimed in one of claims 1 to 8, wherein the inhibition of the enzymes can be reversed by the supply of heat.

10. The composition, kit, use or method as claimed in one of claims 1 to 9, wherein the covalently inhibited enzyme is in each case, independently of one another, DNA polymerase, RNA polymerase, ligase, reverse transcriptase, restriction endonuclease or restriction exonuclease and the non-covalently inhibited enzyme is independently of one another DNA polymerase, RNA polymerase or reverse transcriptase

11. The composition, kit, use or method as claimed in claim 10, wherein both at least one enzyme reversibly inhibited by chemical modification, and at least one polymerase reversibly inhibited by noncovalent binding of a polyanion, is a thermostable DNA polymerase.

12. The composition, kit, use or method as claimed in one of claims 1 to 11, wherein the enzymes are present or are used in a mixture ratio of chemically covalently modified enzyme to polymerase modified by noncovalent binding of a polyanion in a unit mixture ratio from 0.1:1 to 100:1.

13. The composition or kit as claimed in one of claims 1, 2 or 8 to 12, additionally containing further components for a polymerase chain reaction (PCR) or coupled reverse transcription-PCR.

## Revendications

1. Composition renfermant au moins une enzyme inhibée de façon réversible par modification chimique covalente ainsi qu'au moins une polymérase inhibée de façon réversible par liaison non covalente d'un poly-anion.

2. Kit renfermant au moins une enzyme inhibée de façon réversible par modification chimique covalente ainsi qu'au moins une polymérase inhibée de façon réversible par liaison non covalente d'un poly-anion, ou un mélange de telles enzymes.

3. Utilisation d'un mélange d'au moins une enzyme inhibée de façon réversible par modification chimique covalente avec au moins une polymérase inhibée de façon réversible par liaison non covalente d'un poly-anion pour le traitement ou la multiplication de poly-nucléotides.

4. Utilisation conforme à la revendication 3, selon laquelle le traitement ou la multiplication consiste en une amplification spécifique, une ligation, une transcription inverse ou une fragmentation par une ou plusieurs coupures de restriction.

5. Utilisation conforme à la revendication 4, selon laquelle l'amplification spécifique est effectuée par PCR ou PCR après transcription inverse (RT-PCR en une étape).

6. Procédé d'amplification spécifique d'ADN ou d'ARN selon lequel dans au moins une charge réactionnelle sont associées au moins une enzyme inhibée de façon réversible par modification chimique covalente ainsi qu'au moins une polymérase inhibée de façon réversible par liaison non covalente d'un poly-anion.

7. Procédé conforme à la revendication 6, pour l'amplification spécifique d'ADN ou d'ARN, selon lequel l'amplification spécifique est effectuée par PCR ou PCR après transcription inverse (RT-PCR en une étape).

8. Composition, kit, utilisation ou procédé conforme à l'une des revendications 1 à 7, selon lequel les enzymes sont des enzymes thermostables.

9. Composition, kit, utilisation ou procédé conforme à l'une des revendications 1 à 8, selon lequel l'inhibation de l'enzyme peut être effectuée de manière réversible par introduction de chaleur.

10. Composition, kit, utilisation ou procédé conforme à l'une des revendications 1 à 9, selon lequel l'enzyme inhibée par covalence est une ADN polymérase, une ARN polymérase, une Ligase, une transcriptase inverse, une endo-nucléase de restriction ou une exo-nucléase de restriction tandis que le l'enzyme inhibée de façon non covalente est une ADN polymérase, une ARN polymérase ou une transcriptase inverse.

11. Composition, kit, utilisation ou procédé conforme à la revendication 10, selon lequel au moins une enzyme inhibée de façon réversible par modification chimique covalente ainsi qu'au moins une polymérase inhibée de façon réversible par liaison non covalente d'un poly-anion est une ADN polymérase thermostable.

12. Composition, kit, utilisation ou procédé conforme à l'une des revendications 1 à 11, selon lequel les enzymes se présentent ou sont mises en oeuvre dans un rapport de mélange de l'enzyme modifiée par covalence chimique avec la polymérase modifiée par liaison non covalente d'un poly-anion dans un rapport de mélange unitaire de 0,1:1 à 100 :1.

13. Composition ou kit conforme à l'une des revendications 1, 2 ou 8 à 12, renfermant en outre d'autres composants pour permettre une réaction en chaine par polymérase (PCR) ou une RT-PCR en une étape.
